# FASCICULE DE BREVET EUROPEEN

(11) **EP 0 543 722 B1**
(45) Date de publication et mention de la délivrance du brevet: **16.04.1997**
(21) Numéro de dépôt: 92403094.3
(22) Date de dépôt: 18.11.1992
(51) Int. Cl.: C12Q 1/26, G01N 33/14

(54) **Procédé et dispositif de mesure de l'activité Laccase dans les moûts par la méthode à la syringaldazine**
Verfahren und Vorrichtung zum Messen der Laccase-Aktivität in Most mit Hilfe des Syringaldazin-Tests
Method and device for the determination of laccase in must by the syringaldazine test

(30) Priorité: 19.11.1991 FR 9114521
(43) Date de publication de la demande: 26.05.1993
(73) Titulaire: BIO SERAE LABORATOIRES SA, 12400 Saint Affrique (FR)
(72) Inventeur: Angele, Laurent, F-11310 Saissac (FR); Degre, Michel, F-11170 Montolieu (FR)
(74) Mandataire: Cabinet BARRE LAFORGUE & associés

(56) Documents cités:
- EP-A- 0 095 432
- DE-A- 1 698 260
- DE-B- 2 240 661
- CONNAISSANCE DE LA VIGNE ET DU VIN, vol. 18, no. 4, 1984, BORDEAUX FR, pages 237 - 252 D.DUBOURDIEU ET AL. 'Mise au point d'une mésure rapide de l'activité laccase dans les mouts et dans les vins par la méthode à la syringaldazine. Application à l'appreciation de l'etat sanitaire des vendages'
- JOURNAL OF THE SCIENCE OF FOOD AND AGRICULTURE vol. 48, no. 3, 1989, LONDON GB pages 369 - 376 C.GRASSIN ET AL. 'QUANTITATIVE DETERMINATION OF BOTRYTIS LACCASE IN MUSTS AND WINES BY THE SYRINGALDAZINE TEST'
- JOURNAL CHEMICAL TECHNOLOGY AND BIOTECHNOLOGY vol. 41, no. 3, 1988, OXFORD GB pages 243 - 248 N. ZOUARI ET AL. 'A continuous-flow method for the rapid determination of sanitary quality of grape must at industrial scales'

## Description

L'invention concerne un procédé et un dispositif de mesure de l'activité Laccase dans les moûts par la méthode à la syringaldazine.

L'état sanitaire de la vendange, en particulier le niveau de contamination des raisins par la pourriture grise ou Botrytis cinerea, constitue un critère déterminant dans l'appréciation de la qualité de la matière première en vinification.

Malgré cela, et en dépit des progrès dans les connaissances biochimiques acquises sur le métabolisme de Botrytis, aucune méthode analytique d'appréciation de l'état sanitaire de la vendange n'est à ce jour couramment utilisée dans la pratique.

En effet, à l'heure actuelle, l'appréciation de l'état sanitaire de la vendange est effectuée visuellement par notation du pourcentage de baies pourries par rapport au nombre total de baies. Toutefois, cette méthode présente de nombreuses lacunes, d'une part car la mécanisation des vendanges rend de plus en plus difficile la visualisation de la pourriture grise, et d'autre part, car il n'y a pas forcément corrélation exacte entre cette appréciation visuelle et l'activité réelle du Botrytis cinerea qui constitue le seul critère intéressant.

Pour pallier cet inconvénient, des recherches ont été menées en vue de mesurer dans le moût une activité enzymatique exo-cellulaire de Botrytis cinerea, caractéristique du développement de ce champignon dans le raisin et absente dans les baies saines.

Ces recherches ont conduit en premier lieu à la conclusion que la Laccase de Botrytis cinerea satisfait à cette condition, c'est-à-dire est caractéristique du développement de ce champignon, et en second lieu à la mise au point d'une méthode de mesure de l'activité Laccase.

Cette méthode de mesure est connue sous le nom de méthode à la syringaldazine et permet de déterminer par un test simple l'activité Laccase.

Toutefois, si cette méthode est actuellement très fiable au niveau d'un laboratoire, elle ne peut être utilisée en condition réelle de récolte en raison du temps nécessaire pour effectuer le test, incompatible avec les cadences d'arrivée de la vendange.

La présente invention vise à pallier cet inconvénient et a pour premier objectif principal de fournir un procédé et un dispositif de mesure de l'activité Laccase par la méthode à la syringaldazine, adaptés pour effectuer cette mesure de façon entièrement automatisée et rapide (de l'ordre de 2 mn).

Un autre objectif principal de l'invention est de fournir un procédé et un dispositif permettant de détecter toute tentative de fraude visant à fausser le résultat de la mesure de l'activité Laccase.

Un autre objectif de l'invention est de fournir un procédé et un dispositif ne demandant que des interventions humaines très ponctuelles pouvant être effectuées par le personnel déjà en place dans les caves coopératives et dont la fonction est d'effectuer les relevés de pesée et de mesure d'alcool potentiel.

A cet effet, l'invention vise un procédé de mesure de l'activité Laccase dans les moûts par la méthode à la syringaldazine, caractérisé en ce qu'il consiste :
a) à prélever un volume prédéterminé de moût,
b) à clarifier le moût prélevé, et à injecter sous pression, le moût clarifié au travers d'un récipient renfermant une résine apte à adsorber les anthocyanes et les tanins,
c) à récupérer deux doses d'un volume déterminé de moût "décoloré" par ladite résine,
d) à réaliser un échantillon composé d'une des doses de moût et de doses prédéterminées de syringaldazine et de solution tampon,
e) à effectuer à intervalle de temps prédéterminé deux mesures colorimétriques de l'échantillon à une longueur d'onde où la couleur rouge absorbe,
f) à délivrer des signaux représentatifs des densités optiques relevées lors des deux mesures colorimétriques,
g) à calculer l'activité Laccase en fonction de la différence entre les résultats des deux mesures colorimétriques,
h) à délivrer vers des moyens d'affichage un signal représentatif de l'activité Laccase calculée,
i) et en parallèle par rapport aux étapes d), e), f), g) et h) précitées,
j) à réaliser un échantillon composé de l'autre dose de moût décoloré, et d'une dose prédéterminée de solution iodée, et à vérifier la présence éventuelle de dioxyde de soufre dans l'échantillon précité,
k) et à délivrer un signal d'inhibition du résultat de la mesure de l'activité Laccase si la concentration en dioxyde de soufre est supérieure à un seuil prédéterminé.

Ce procédé permet, en premier lieu, d'apprécier objectivement et dès son arrivée sur le quai de réception, le degré d'attaque de la vendange par Botrytis cinerea et ainsi :
. d'adopter un processus technologique de vinification,
. d'optimiser la qualité des produits issus,
. et de rémunérer en conséquence les adhérents.

En second lieu, la détection du taux de dioxyde de soufre éventuel par une mesure "tout ou rien" effectuée en temps masqué par rapport au dosage de l'activité Laccase, permet de se garantir contre tout risque de fraude. En effet, la présence d'un taux donné de dioxyde de soufre a pour résultat de fausser la mesure de l'activité Laccase et peut donc conduire à classer comme saine une vendange altérée.

Par ailleurs, ce dosage de l'activité Laccase ne requiert que quelques minutes (de l'ordre de 2 mn) du fait notamment que la décoloration du moût par la résine se fait très rapidement par injection de ce moût sous pression à travers ladite résine.

Selon une autre caractéristique de l'invention (j) :
j1) l'on effectue une mesure colorimétrique à blanc, d'étalonnage, à une longueur d'onde où la couleur bleue absorbe,
j2) l'on réalise un échantillon composé d'une dose prédéterminée de moût décoloré et de doses prédéterminées d'un indicateur coloré, tel que du thiodène, d'un acide tel que l'acide sulfurique, et d'iode,
j3) l'on effectue une mesure colorimétrique de l'échantillon à la longueur d'onde où la couleur bleue absorbe,
j4) l'on mesure la valeur correspondant à la différence de densité optique entre les deux mesures, et
(k) l'on délivre un signal d'inhibition du résultat de la mesure de l'activité Laccase si la valeur mesurée est supérieure à un seuil prédéterminé.

L'invention s'étend à un dispositif de mesure de l'activité Laccase comprenant :
- des moyens de prélèvement adaptés pour collecter une quantité prédéterminée de moût,
- des moyens de clarification du moût prélevé,
- un récipient doté d'une entrée de fluide et d'une sortie de fluide et renfermant une résine apte à adsorber les anthocyanes et les tanins, ledit récipient étant apte à laisser s'écouler le moût par la sortie de fluide et à retenir la résine,
- des moyens de mise en pression adaptés pour injecter sous pression le moût clarifié au travers du récipient renfermant la résine,
- des moyens de stockage de produits chimiques contenant notamment de la syringaldazine, une solution tampon, et une solution iodée,
- des premiers moyens de dosage adaptés pour réaliser un premier échantillon constitué de doses prédéterminées de moût décoloré, de syringaldazine et de solution tampon,
- des deuxièmes moyens de dosage adaptés pour réaliser un deuxième échantillon constitué de doses prédéterminées de moût décoloré et de solution iodée,
- des moyens de mesure colorimétrique adaptés pour effectuer des mesures de densité optique du premier échantillon, à une longueur d'onde où la couleur rouge absorbe,
- des moyens de mesure adaptés pour détecter la présence éventuelle de dioxyde de soufre dans le deuxième échantillon,
- des moyens de transmission aptes à transmettre des signaux représentatifs des résultats des mesures effectuées par les moyens de mesure précitées,
- des moyens de canalisation adaptés pour assurer le transfert du moût et des échantillons vers les moyens de mesure,
- et une unité centrale reliée aux moyens de transmission et adaptée, d'une part, pour activer successivement les moyens de prélèvement, les moyens de mise en pression, les moyens de dosage et les moyens de mesure et d'autre part, pour :
- commander à intervalle de temps déterminé, deux mesures colorimétriques du premier échantillon et calculer l'activité Laccase à partir des deux signaux délivrés par les moyens de mesure colorimétrique, puis délivrer un signal représentatif de ladite activité Laccase vers des moyens d'affichage,
- délivrer un signal d'inhibition de la mesure de l'activité Laccase, si la concentration en dioxyde de soufre dans le deuxième échantillon est supérieure à une valeur prédéterminée.

Selon une autre caractéristique de l'invention :
- les moyens de stockage de produits chimiques contiennent également un indicateur coloré tel que du thiodène, et un acide tel que l'acide sulfurique,
- les deuxièmes moyens de dosage sont adaptés pour réaliser un échantillon constitué de doses prédéterminées de moût décoloré, d'indicateur coloré, d'acide et de solution iodée,
- les moyens de mesure pour la détection de dioxyde de soufre sont des moyens de mesure colorimétrique à une longueur d'onde où la couleur bleue absorbe,
- l'unité centrale est adaptée pour commander une première mesure colorimétrique à blanc, d'étalonnage, des moyens de mesure colorimétrique, commander le transfert de l'échantillon vers lesdits moyens de mesure, commander une deuxième mesure colorimétrique, et calculer la concentration en dioxyde de soufre par mesure de la valeur correspondant à la différence entre les deux mesures effectuées.

De plus, selon un mode de réalisation préférentiel :
- les moyens de prélèvement comprennent des moyens de pompage aptes à amener le long d'une canalisation de pompage une quantité prédéterminée de moût à l'intérieur d'une cuve de stockage dotée d'une ouverture de remplissage et d'une ouverture d'évacuation,
- les moyens de clarification comprennent une crépine de filtration disposée au niveau de l'ouverture d'évacuation de la cuve de stockage.

En outre, et de façon avantageuse, la cuve de stockage et la canalisation de pompage sont reliées à des moyens d'alimentation en eau de rinçage et en air de séchage aptes à permettre le lavage et le séchage desdites cuve et canalisation.

Par ailleurs, selon une autre caractéristique de l'invention, les premier et deuxième moyens de dosage comprennent un doseur de moût doté de deux chambres de dosage de volume prédéterminé, et des moyens de déplacement desdites chambres entre une position de remplissage par le moût décoloré sortant du récipient, et une position de vidange vers les moyens de mesure respectifs de chaque échantillon.

Ce doseur comporte, de plus, préférentiellement, pour chaque échantillon :
- un fourreau présentant deux tronçons de sections différentes : un tronçon amont doté d'une ouverture de communication avec un récipient, et un tronçon aval de section supérieure à celle du tronçon amont, doté d'une ouverture de communication avec les moyens de mesure,
- un tiroir de section conjuguée de celle du tronçon amont du fourreau, comportant un tronçon intermédiaire de section inférieure à sa section courante, formant une chambre de dosage annulaire à l'intérieur dudit tronçon amont,
- des moyens d'étanchéité solidaires du tiroir et disposés de part et d'autre de la chambre de dosage,
- les moyens de déplacement étant adaptés pour déplacer les tiroirs entre une position de remplissage où les chambres de dosage sont disposées dans les tronçons amont desdits fourreaux en regard de l'ouverture de ces derniers, et une position de vidange où lesdites chambres de stockage sont disposées dans les tronçons aval de façon à permettre l'évacuation du moût au travers des ouvertures desdits tronçons aval.

En outre, ce doseur comporte, avantageusement, un bloc d'évacuation du moût comprenant :
- deux réservoirs agencés pour être alimentés respectivement par les premier et deuxième moyens de dosage en réactifs, et dotés d'une sortie d'évacuation des échantillons vers les moyens de mesure correspondants,
- deux conduits agencés pour venir en continuité chacun d'un fourreau, de façon à relier l'ouverture du tronçon aval de ce dernier à un réservoir.

Le dispositif selon l'invention comprend, par ailleurs, selon une autre caractéristique :
- des moyens de déplacement en translation des moyens de mesure aptes à les déplacer entre une position avancée de remplissage à l'aplomb du bloc d'évacuation du doseur, et une position reculée de mélange où les ouvertures de remplissage sont fermées par un obturateur fixe,
- des moyens de rotation des moyens de mesure autour de leur axe de translation.

Cette disposition permet de procéder au mélange des produits formant chaque échantillon avant les mesures. De plus, elle permet, avantageusement, de laver les enceintes de mesure à l'aide de moyens de rinçage et de séchage desdites enceintes de mesure, disposés de façon à assurer le lavage et le séchage de ces dernières dans leur position avancée, après une rotation de 180° par rapport à leur position de remplissage.

Selon une autre caractéristique, le dispositif selon l'invention comprend, en outre :
- un magasin apte à loger une pluralité de récipients,
- des moyens de préhension individuelle d'un récipient dans le magasin,
- et des moyens de déplacement des moyens de préhension aptes à déplacer ces derniers entre une position de préhension d'un récipient, et une position où ledit récipient se trouve interposé entre les moyens de prélèvement et les moyens de dosage.

De plus, de façon préférentielle, ce dispositif comprend un récipient vide porté par les moyens de déplacement et agencé pour se trouver interposé entre la cuve de stockage et le doseur, dans la position de préhension des moyens de déplacement, de façon à permettre un lavage et un séchage du doseur par l'intermédiaire des moyens d'alimentation en eau de rinçage et en air de séchage de ladite cuve.

Un tel dispositif permet de minimiser le nombre d'interventions à effectuer par l'opérateur, à sa plus simple expression puisque ces interventions se limitent à :
. déclencher le cycle, par exemple, par une simple pression effectuée sur un bouton, engendrant le démarrage de ce cycle qui s'effectue jusqu'à l'affichage des résultats,
. à approvisionner le magasin de récipients,
. à renouveler périodiquement les réactifs.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins qui font partie intégrante de la présente description :

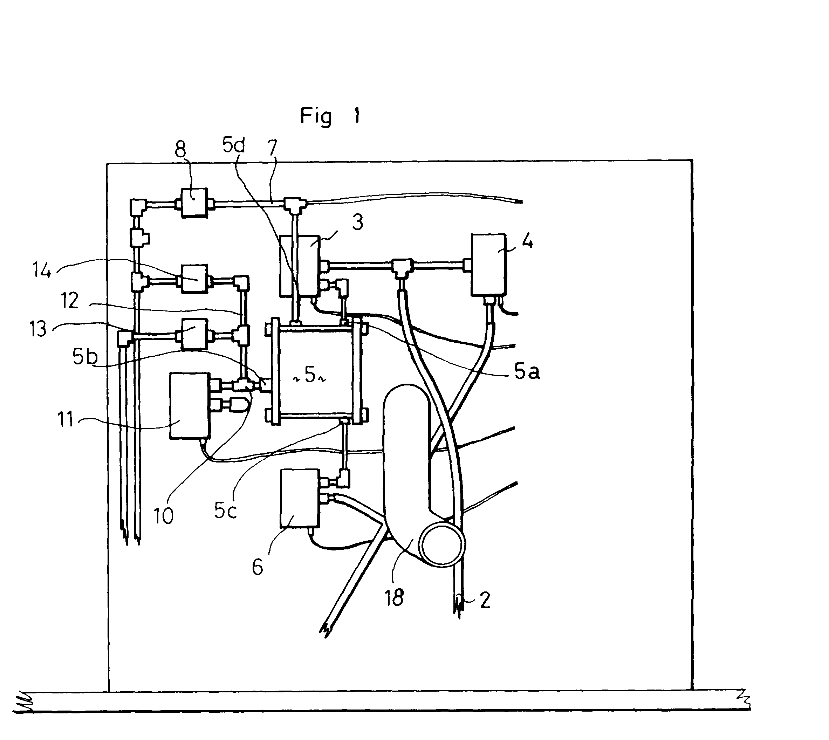

- la figure 1 est une vue de face schématique, des moyens de prélèvement du moût, et des moyens de rinçage et de séchage desdits moyens de prélèvement,

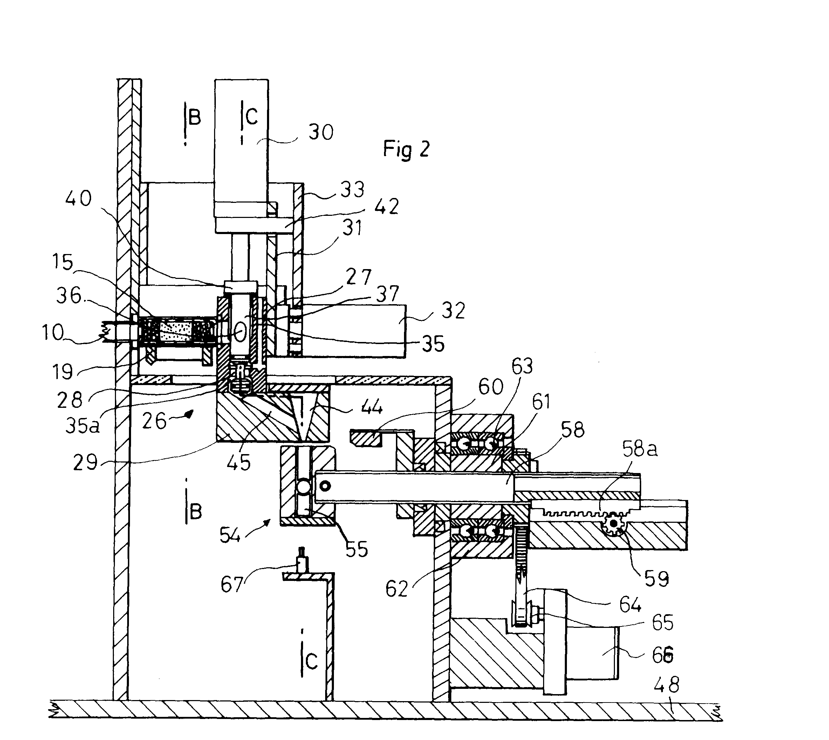

- la figure 2 est une coupe transversale par un plan vertical en escalier A, des moyens de dosage et de mesure d'un dispositif conforme à l'invention,

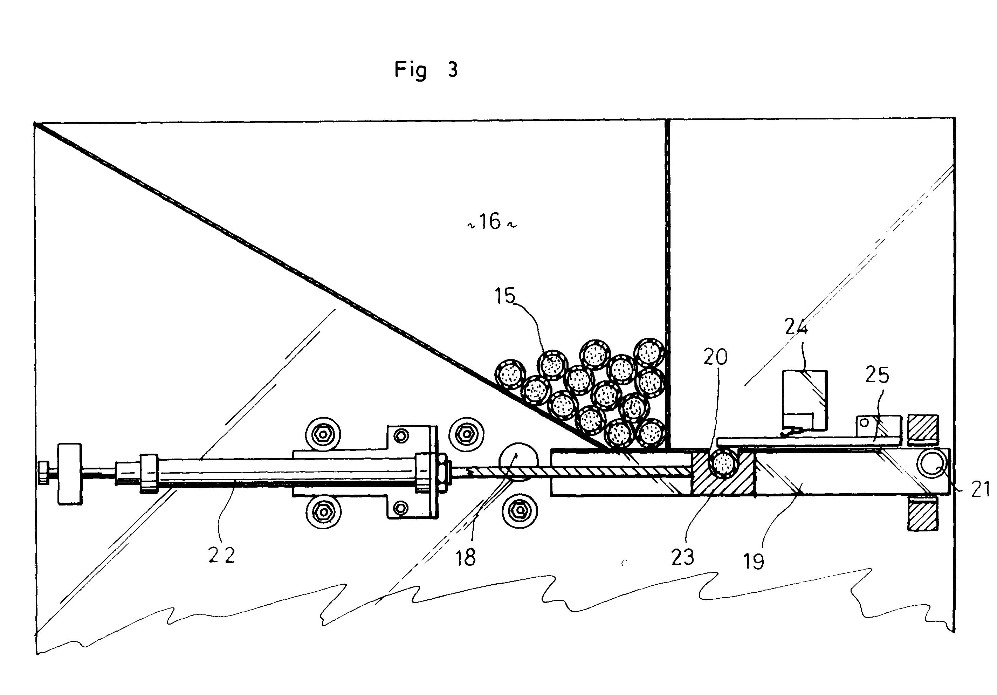

- la figure 3 en est une coupe longitudinale par un plan vertical B, représentant le magasin, les moyens de préhension et de déplacement des récipients,

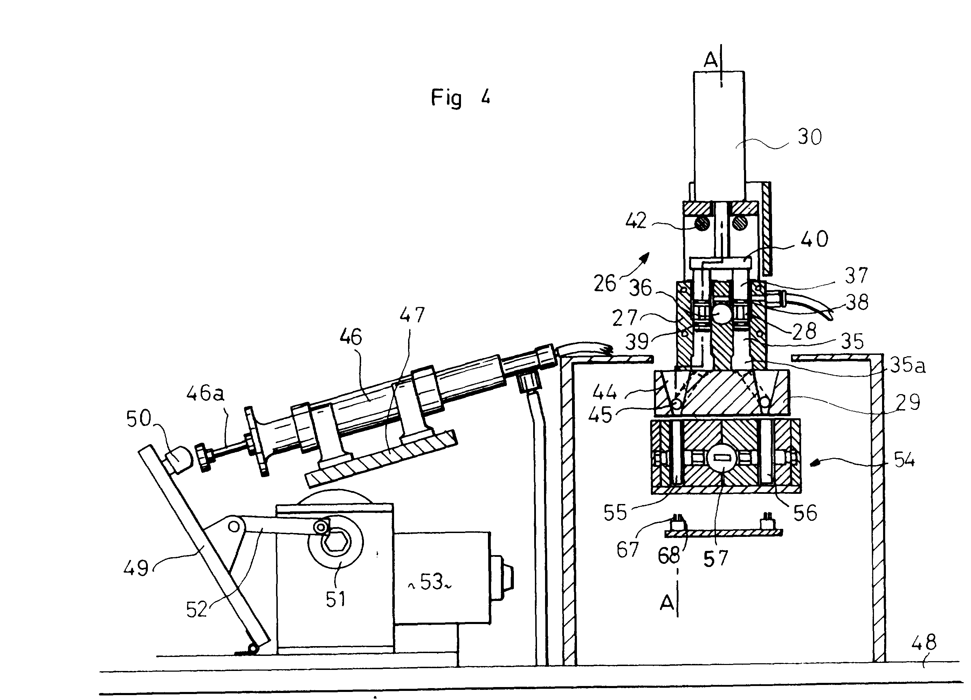

- la figure 4 en est une coupe transversale par un plan vertical en escalier C,

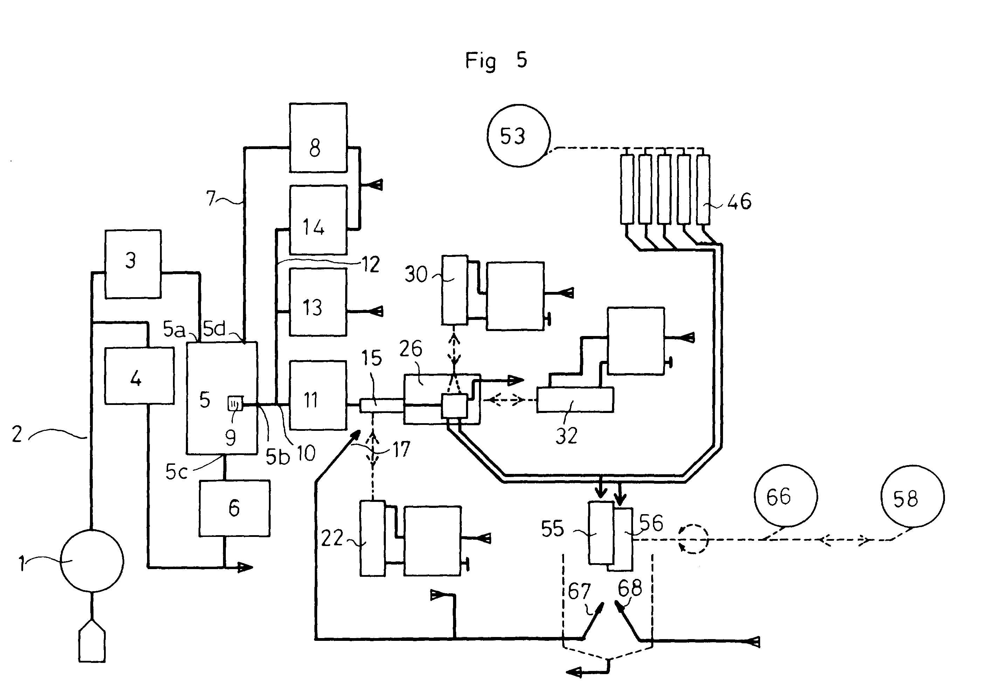

- la figure 5 est un schéma représentatif de l'ensemble des éléments constitutifs d'un dispositif conforme à l'invention,
- et la figure 6 est un logigramme de fonctionnement de ce dispositif.

Le dispositif représenté aux figures est destiné à effectuer un dosage entièrement automatisé et rapide (de l'ordre de 2 mn) de l'activité Laccase sur les moûts, en application de la méthode à la syringaldazine. Ce dispositif permet donc, à partir d'une analyse hautement spécifique d'apprécier objectivement le degré d'attaque de la matière première par le Botrytis cinerea.

En outre, ce dispositif est adapté pour déterminer le taux de dioxyde de soufre libre par une mesure "tout ou rien" (comparaison par rapport à un seuil fixé, par exemple à 10 mg/litre de dioxyde de soufre libre) et ainsi de détecter toute fraude. En effet, la présence d'un taux donné de dioxyde de soufre a pour résultat de fausser la mesure de l'activité Laccase et peut donc conduire à classer comme saine une vendange altérée.

Le dispositif est, par ailleurs, conçu pour réaliser cette analyse de dioxyde de soufre et ce dosage de l'activité Laccase à partir d'un échantillon de moût de raisin, selon un cycle se décomposant en six étapes essentielles :
- le prélèvement du moût de raisin,
- la gestion d'un magasin d'approvisionnement de cartouches contenant de la P.V.P.P.,
- la filtration et la "décoloration" du moût filtré dans les cartouches à l'aide d'un circuit d'air sous pression qui force l'air à passer à travers la résine,
- le dosage de l'activité Laccase réalisé dans un module d'analyse par spectrophotométrie,
- l'analyse de dioxyde de soufre réalisée dans un deuxième module d'analyse par spectrophotométrie,
- et enfin, l'édition du résultat assurée par exemple par une micro-imprimante et/ou un afficheur digital.

Pour réaliser la première étape de prélèvement du moût de raisin, le dispositif comprend une pompe 1 refoulant dans une canalisation de pompage 2 sur laquelle sont interposées une électrovanne d'alimentation 3 et une électrovanne de mise à l'égout 4.

L'électrovanne de vidange 4 permet de raccorder la pompe 1 directement à l'égout tandis que l'électrovanne d'alimentation 3 est raccordée à l'ouverture de remplissage 5a d'une cuve de stockage 5 dotée par ailleurs d'une ouverture d'évacuation 5b, et d'une ouverture de vidange 5c raccordée à l'égout par l'intermédiaire d'une canalisation sur laquelle est interposée une électrovanne de vidange 6.

Cette cuve de stockage 5 comporte, en outre, en partie supérieure, une entrée 5d d'alimentation en air comprimé reliée à une source d'air comprimé (non représentée et pouvant consister en une source externe au dispositif ou une source livrée avec ce dernier) par l'intermédiaire d'une canalisation 7 obturée par une électrovanne 8.

Au niveau de sa sortie d'évacuation 5b, la cuve de stockage comporte une crépine de filtration 9 logée à l'intérieur de ladite cuve.

Cette sortie d'évacuation 5b est, par ailleurs, connectée à une conduite 10 dotée d'une électrovanne 11. Sur cette conduite 10 viennent, en outre, se raccorder une canalisation 12 dotée d'un piquage relié à une source d'alimentation en eau de rinçage par l'intermédiaire d'une électrovanne 13, et d'un piquage relié à la source d'air comprimé par l'intermédiaire d'une électrovanne 14.

Le moût aspiré par la pompe 1 est délivré vers la cuve de stockage 5, et est ensuite filtré par la crépine 9 et refoulé dans la canalisation 10 sous pression d'air comprimé admis au niveau de l'entrée 5d de ladite cuve.

Le moût ainsi refoulé sous pression passe au travers d'une cartouche 15 renfermant un mélange en parties égales de résine P.V.P.P. et de sable et est délivré vers l'entrée d'un doseur comportant deux chambres de dosage.

Chacune de ces cartouches comporte deux extrémités, dotées de pastilles percées d'une perforation centrale apte à permettre une circulation du moût et, interposées entre lesdites pastilles et selon le sens d'écoulement du moût, au moins deux couches de matériau de filtration de porosité décroissante, une épaisseur de résine constituée de polyvinylpolypyrrolidone additionnée de sable, et au moins une couche de matériau apte à retenir la résine et à laisser s'écouler le moût.

Chaque cartouche 15 assure donc, d'une part, une filtration additionnelle du moût et, d'autre part, la décoloration de ce dernier par la résine.

Afin d'assurer le remplacement de la cartouche 15 après chaque mesure, le dispositif comprend un magasin 16 de stockage desdites cartouches, des moyens de préhension d'une des cartouches logée dans ce magasin 16, et des moyens de déplacement aptes à amener ladite cartouche soit en regard de la canalisation 10, soit en regard d'une buse d'air comprimé 17 permettant de l'éjecter après usage dans un tube 18 de récupération des cartouches usagées.

En premier lieu, le magasin de stockage 16 présente la forme d'un entonnoir de forme triangulaire et de largeur constante égale à la longueur des cartouches 15. Cet entonnoir comporte, en partie basse, une ouverture permettant l'évacuation de deux ou trois cartouches.

Les moyens de préhension comprennent, quant à eux, deux rails 19 horizontaux et parallèles s'étendant sous l'ouverture du magasin 15, et dotés, en regard, de deux encoches 20 de formes conjuguées de la section d'une cartouche 15 aptes à loger une cartouche se présentant au droit de l'ouverture du magasin 16.

Ces deux rails 19 sont, en outre, reliés vers leur extrémité, par un tube vide 21 de même diamètre que celui d'une cartouche, s'étendant entre deux orifices de diamètre conjugué, ménagés dans chacun desdits rails. Ce tube 21 est disposé de façon à se trouver en regard de la canalisation 10 lorsque la cartouche 15 usagée se trouve en regard de la buse 17 en vue de son éjection dans le tube de récupération 18.

En outre, les moyens de déplacement comprennent un vérin 22 agencé horizontalement et dont la tige est solidarisée à une entretoise 23 reliant les rails 19 au droit des encoches 20. Ce vérin 22 est en plus asservi à des moyens de détection de fin de course comportant un contact 24 à sabot, et une lame souple 25 articulée vers une de ses extrémités, et agencée pour pivoter vers le haut et activer le contact 24 lorsqu'elle rencontre une cartouche 15 ou le tube vide 21.

Comme indiqué plus haut, le dispositif comprend, en aval de la cartouche 15 et dans le sens d'écoulement du moût, un doseur 26 comportant un bloc de dosage 27 doté de deux chambres de dosage telles que 28, un bloc 29 d'évacuation du moût, et des moyens 30 de déplacement des chambres de stockage 28 entre une position de remplissage et une position d'évacuation.

En outre, ce doseur 26 est porté par un bâti comportant une paroi verticale 31 solidarisée sur l'extrémité de la tige d'un vérin 32 agencé horizontalement, et dont le corps est lui-même fixé sur une paroi verticale fixe 33.

Ce vérin 32 a pour but de déplacer le doseur 26 selon une translation horizontale de façon à permettre de le reculer en vue du déplacement des rails 29 et de l'avancer une fois qu'une cartouche 15 ou le tube 21 se trouve en position en regard dudit doseur.

Le doseur 26 comprend, en premier lieu, un bloc de dosage 27 percé longitudinalement de deux fourreaux verticaux 35 dans chacun desquels débouche radialement un conduit horizontal 36 agencé pour permettre au moût sortant de la cartouche 15 de se déverser dans lesdits fourreaux.

En outre, chacun de ces fourreaux 35 comporte, en partie basse, un tronçon 35a de diamètre supérieur à son diamètre courant.

Le doseur 26 comprend, de plus, deux tiroirs 37 de diamètre conjugué du diamètre courant des fourreaux 35 agencés pour venir se loger dans lesdits fourreaux. Chacun de ces tiroirs 37 comporte, en outre, un tronçon intermédiaire de diamètre inférieur à son diamètre courant, délimitant à l'intérieur du fourreau 35 une chambre de dosage annulaire 28 d'une capacité de 1 cm³.

De plus, deux joints d'étanchéité annulaires 38, 39 portés par chaque tiroir 37 de part et d'autre de la chambre de dosage 28, permettent d'assurer l'étanchéité de cette dernière lorsqu'elle se trouve dans le tronçon supérieur du fourreau 35.

Les deux tiroirs 37 sont, par ailleurs, fixés sous une platine horizontale 40 disposée au-dessus du bloc de dosage 34. Cette platine 40 est elle-même solidaire de l'extrémité de la tige d'un vérin 30 agencé verticalement au-dessus du bloc de dosage 34, et dont le corps est porté par la paroi verticale 31.

Un tel vérin 30 est adapté pour déplacer les tiroirs 37 de façon que les chambres de dosage 28 viennent, soit dans une position haute de remplissage où elles se trouvent en communication avec le conduit 36, soit dans une position basse d'évacuation où lesdites chambres se trouvent à l'intérieur du tronçon bas 35a, de diamètre supérieur, du fourreau 35, autorisant l'évacuation du moût par gravité.

Par ailleurs, deux axes de butée horizontaux 42, fixés sur la paroi verticale 33 et s'étendant au travers d'orifices ménagés dans la paroi verticale 31, sont disposés de façon à servir de butée haute à la platine 40 lorsque le doseur 26 se trouve accolé à une cartouche 15, cette butée haute correspondant à la position de remplissage des chambres de dosage 28.

Par contre, le tube vide 21 et l'extrémité des rails 29 sont adaptés pour que le doseur 26 se trouve dans une position plus avancée lorsqu'il se trouve accolé audit tube, que lors de sa position accolée contre une cartouche 15, de façon que les axes de butée 42 soient inopérants, c'est-à-dire de façon que la platine 40 se déplace verticalement devant l'extrémité de ces axes.

Cet agencement permet de remonter les chambres de dosage 28 au-dessus du conduit 36 et, par conséquent, autorise le lavage et le séchage du tronçon inférieur des fourreaux 35, tel que décrit plus loin.

Le doseur 26 comporte, enfin, un bloc d'évacuation 29 fixé sous le bloc de dosage 27 et de section horizontale supérieure à celle dudit bloc de dosage. Ce bloc d'évacuation 29 comporte, en premier lieu, deux réservoirs 44 en forme d'entonnoirs à axes verticaux, débouchant respectivement au droit des faces supérieures et inférieures dudit bloc.

Il comporte, en outre, deux conduits obliques 45 reliant chacun l'extrémité basse d'un fourreau 35 à un réservoir 44 et autorisant l'écoulement du moût par gravité vers lesdits réservoirs.

Ces réservoirs 44 sont destinés à être alimentés en réactifs au moyen de distributeurs 46 connus sous le nom de pipettes de dosage, renfermant chacune un réactif, et réglées de façon à délivrer chacune une dose prédéterminée de réactif lors de l'actionnement du piston 46a desdites pipettes.

Ces pipettes, au nombre de cinq, comportent chacune une sortie de fluide reliée à un des réservoirs 44, et une entrée de fluide alimentée à partir de moyens de stockage (non représentés).

Ces moyens de stockage consistent en cinq flacons, un par pipette, renfermant respectivement une solution tampon constituée d'acétate de sodium 0,1 M à pH 5, de la syringaldazine à 60 mg/litre, de l'acide sulfurique, du thiodène, et une solution iodée composée d'un mélange d'iodure et d'iodate de potassium.

Ces pipettes sont portées par une plaque inclinée 47 disposée à distance au-dessus d'une plate-forme 48 formant la plate-forme de base du dispositif. L'actionnement des pistons 46a de ces pipettes est réalisé au moyen d'une plaque 49 articulée sur la plate-forme 47 et dotée, en regard de chaque extrémité de piston 46a, d'un doigt 50 apte à venir en contact avec ledit piston et à le repousser lors d'un pivotement de la plaque articulée 49.

Ce pivotement de la plaque est assuré par un système manivelle 51/bielle 52 articulé à mi-hauteur de ladite plaque et actionné par un moteur 53.

Le dispositif comprend, par ailleurs, un bloc de mesure 54 portant deux enceintes de mesure 55 et 56 intégrées dans ledit bloc et débouchant au niveau de la face supérieure de ce dernier. Ce bloc de mesure 54 renferme, en outre, une diode électroluminescente apte à émettre à une longueur d'onde de 665 nm vers une des enceintes de mesure 56 permettant la mesure de la concentration en dioxyde de soufre.

Ce bloc de mesure 54 renferme enfin une lampe à incandescence 57 associée à un filtre apte à permettre l'émission à une longueur d'onde de 530 nm vers l'autre enceinte de mesure 55 permettant les mesures de l'activité Laccase.

Cette lampe à incandescence 57 est, en outre, associée à des moyens de régulation de son intensité aptes à permettre de maintenir les échantillons renfermés dans les enceintes de mesure 55, 56, à une température de 37° C environ.

Ce bloc de mesure 54 est porté par un axe 58, dont l'extrémité 58a opposée au bloc présente la forme d'une crémaillère coopérant avec un pignon 59. Ce système pignon 59/crémaillère 58a est apte à engendrer un déplacement horizontal dudit axe et donc du bloc de mesure entre une position avancée où les enceintes de mesure 55, 56 se trouvent sous les réservoirs 44 permettant le remplissage desdites enceintes par gravité, et une position reculée où les enceintes de mesure 55, 56 se trouvent coiffées d'un obturateur 60 apte à assurer l'étanchéité de ces dernières.

Le bloc de mesure 54 est, par ailleurs, associé à des moyens de mise en rotation dudit bloc autour d'un axe horizontal.

Ces moyens de mise en rotation comprennent un rotor 61 solidaire d'une couronne porte-obturateur 60, traversé par l'axe 58 et monté rotatif dans une cage fixe 62 par l'intermédiaire de roulements à billes 63.

Ce rotor 61 est, en outre, solidaire d'un pignon (non représenté) entraîné en rotation au moyen d'une courroie sans fin 64 entraînée par un pignon moteur 65 solidaire de l'arbre d'un moteur 66.

Enfin, le bloc de mesure est associé à deux ensembles de rinçage et de séchage destinés à coopérer chacun avec une enceinte de mesure 55, 56.

Ces deux ensembles comprennent chacun une buse 67 de soufflage d'air comprimé, et un injecteur d'eau 68 disposés de façon à se trouver sous le bloc de mesure 54 à l'aplomb des enceintes 55, 56, dans la position avancée dudit bloc et après rotation de 180° de ce dernier visant à assurer la vidange desdites enceintes.

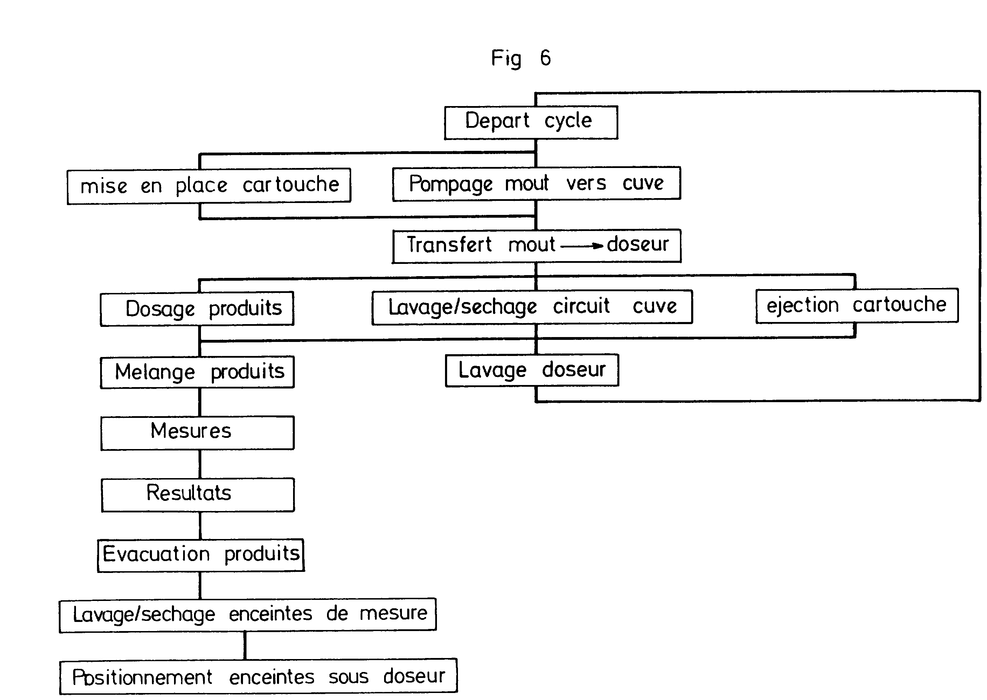

Le logigramme de fonctionnement du dispositif décrit ci-dessus est représenté à la figure 6.

En premier lieu, deux actions sont menées en parallèle, à savoir la mise en place d'une nouvelle cartouche 15 et le prélèvement de moût.

La mise en place d'une cartouche nécessite dans un premier temps de positionner le doseur 26 dans sa position reculée par rétraction du vérin 32. Le vérin 22 est alors déployé jusqu'à ce que la cartouche parvienne en regard de la canalisation 10, cette position étant détectée par le contact 24. Le doseur 26 est alors avancé par déploiement du vérin 32 de façon que la canalisation 10, la cartouche 25 et le conduit 36 soient en liaison étanche.

Le prélèvement du moût consiste, en premier lieu, à enclencher la pompe 1, l'électrovanne d'alimentation 3 étant fermée et l'électrovanne de vidange 4 étant ouverte, de façon à chasser le liquide subsistant dans la canalisation de pompage 2.

Ensuite, l'électrovanne d'alimentation 3 est ouverte, et l'électrovanne de vidange fermée de façon à délivrer le moût vers la cuve de stockage 5.

Une fois la pompe 1 arrêtée, l'électrovanne 11 est ouverte et, après une temporisation, l'électrovanne 8 d'admission d'air comprimé est également ouverte entraînant l'injection du moût sous pression au travers de la cartouche 15 vers les chambres de dosage 28, le doseur 26 étant en position haute. Enfin, après une temporisation, l'électrovanne 11 est fermée.

Par la suite, trois séries d'actions sont menées en parallèle.

La première de ces séries consiste, dans un premier temps, à doser les différents produits. Ce dosage consiste, en ce qui concerne le moût, à amener le doseur 26 en position basse au moyen du vérin 30 de façon à permettre aux deux doses de moût de s'écouler par gravité vers les enceintes de mesure 55, 56 disposées à l'aplomb du bloc d'évacuation 29.

Simultanément, les pipettes 46 sont activées par l'intermédiaire de la plaque 49 et du système manivelle 51/bielle 52.

En ce qui concerne l'échantillon destiné à permettre la mesure de l'activité Laccase, celui-ci est composé d'un centimètre cube de moût décoloré, de 0,6 centimètre cube de syringaldazine à 60 mg/litre et de 1,4 centimètre cube d'acétate de sodium 0,1 M à pH 5.

L'échantillon destiné à la mesure de concentration en dioxyde de soufre comporte, quant à lui, un centimètre cube de moût décoloré, 1 centimètre cube d'acide sulfurique, 0,5 centimètre cube de thiodène, et 0,5 centimètre cube d'un mélange d'iodure et d'iodate de potassium.

Il est à noter que, préalablement au remplissage de l'enceinte de mesure 56 recevant l'échantillon pour le dosage de dioxyde de soufre, il est effectué une première mesure à blanc d'étalonnage.

Après une temporisation permettant l'écoulement des produits dans les enceintes de mesure 55, 56, le bloc de mesure 54 est reculé jusqu'à ce que lesdites enceintes soient coiffées des obturateurs 60.

Le moteur 66 est alors activé alternativement selon un sens de rotation puis selon un sens inverse, de façon à permettre un parfait mélange des produits.

Les mesures de l'activité Laccase et de la détection de présence de dioxyde de soufre sont alors effectuées puis les résultats affichés. Ces mesures consistent, pour l'activité Laccase, à effectuer deux mesures successives par exemple 45 s et 1,15 mn après l'arrêt du mélange. Pour la mesure de dioxyde de soufre, elles consistent à effectuer une seule mesure.

L'étape suivante, d'évacuation des produits est réalisée en faisant pivoter le bloc de mesure 54 de 180° puis en faisant avancer ce dernier au moyen du système crémaillère 58a/pignon 59.

Une fois les enceintes 56, 57 vides, elles sont rincées à l'eau puis séchées à l'air comprimé par l'ensemble buse 67/injecteur 68.

Enfin, le bloc de mesure 54 est ramené sous le bloc d'évacuation 29 dans sa position d'attente d'arrivée de nouveaux échantillons.

La deuxième série d'actions consiste, dans un premier temps, à assurer la vidange, le lavage et le séchage de la cuve de stockage 5.

A cet effet, en premier lieu, l'électrovanne de vidange 6 est ouverte et le moût est chassé de la cuve 5 vers l'égout sous pression d'air comprimé.

Ensuite l'électrovanne 13 d'amenée d'eau est ouverte entraînant le lavage de la cuve 5. L'électrovanne de vidange 6 est alors fermée et les électrovannes d'alimentation 3 et de mise à l'égout 4 ouvertes assurant le lavage de la totalité de la cuve 5 et des canalisations reliant ces électrovannes.

Enfin, les mêmes opérations que celles de lavage sont réalisées au moyen d'air de séchage après ouverture de l'électrovanne 14.

Dans un deuxième temps, on procède au lavage du doseur 26.

Ce lavage est réalisé en avançant le doseur 26 au contact du tube vide 21. Ce lavage est assuré par ouverture de l'électrovanne d'amenée d'eau 13, l'électrovanne 11 interposée sur la conduite 10 étant ouverte. Il est effectué, en premier lieu, le doseur 26 étant en haut et les chambres de dosage 28 se trouvant donc au-dessus de la conduite 36, puis en second lieu, le doseur 26 étant en bas de façon à laver les chambres de dosage 28.

Le rinçage par air comprimé est réalisé ensuite en réalisant les mêmes étapes que précédemment après ouverture de l'électrovanne 14 d'amenée d'air.

Enfin, la troisième série d'actions est destinée à l'éjection de la cartouche 15 précédemment utilisée. Cette opération consiste à amener ladite cartouche en regard de la buse d'air comprimé 17, position qui correspond, en outre, à un positionnement du doseur 26 en regard du tube vide 21 permettant le lavage dudit doseur.

Une fois la cartouche 15 en regard de la buse 17, un jet d'air entraîne l'éjection de cette dernière dans le tube de récupération 18.

Toutes les opérations ci-dessus décrites sont réalisées dans un laps de temps de l'ordre de 2 mn et permettent, entre deux mesures, de laver et de rincer la totalité de l'installation de façon qu'une mesure ne soit pas altérée par la présence de moût ou de produits utilisés pour la mesure précédente.

## Revendications

1. Procédé de mesure de l'activité Laccase dans les moûts par la méthode à la syringaldazine, caractérisé en ce qu'il consiste :
a) à prélever un volume prédéterminé de moût,
b) à clarifier le moût prélevé, et à injecter sous pression, le moût clarifié au travers d'un récipient (15) renfermant une résine apte à adsorber les anthocyanes et les tanins,
c) et à récupérer deux doses d'un volume déterminé de moût "décoloré" par ladite résine,
d) à réaliser un échantillon composé d'une des doses de moût et de doses prédéterminées de syringaldazine et de solution tampon,
e) à effectuer dans un intervalle de temps prédéterminé deux mesures colorimétriques de l'échantillon à une longueur d'onde où la couleur rouge absorbe,
f) à délivrer des signaux représentatifs des densités optiques relevées lors des deux mesures colorimétriques,
g) à calculer l'activité Laccase en fonction de la différence entre les résultats des deux mesures colorimétriques,
h) à délivrer vers des moyens d'affichage un signal représentatif de l'activité Laccase calculée,
i) et en parallèle par rapport aux étapes d), e), f), g) et h) précitées,
j) à réaliser un échantillon composé de l'autre dose de moût décoloré, et d'une dose prédéterminée de solution iodée, et à vérifier la présence éventuelle de dioxyde de soufre dans l'échantillon précité,
k) et à délivrer un signal d'inhibition du résultat de la mesure de l'activité Laccase si la concentration en dioxyde de soufre est supérieure à un seuil prédéterminé.

2. Procédé selon la revendication 1 dans lequel on effectue les mesures colorimétriques à une longueur d'onde égale à 530 nm.

3. Procédé selon l'une des revendications 1 ou 2 caractérisé en ce qu'à l'étape (j) :
j1) l'on effectue une mesure colorimétrique à blanc, d'étalonnage, à une longueur d'onde où la couleur bleue absorbe,
j2) l'on réalise un échantillon composé d'une dose prédéterminée de moût décoloré et de doses prédéterminées d'un indicateur coloré, tel que du thiodène, d'un acide tel que l'acide sulfurique, et d'iode,
j3) l'on effectue une mesure colorimétrique de l'échantillon à la longueur d'onde où la couleur bleue absorbe,
j4) l'on mesure la valeur correspondant à la différence de densité optique entre les deux mesures, et
(k) : l'on délivre un signal d'inhibition du résultat de la mesure de l'activité Laccase si la valeur mesurée est supérieure à un seuil prédéterminé.

4. Procédé selon la revendication 3 caractérisé caractérisé en ce qu'à l'étape (j) l'on effectue les mesures colorimétriques à une longueur d'onde égale à 665 nm.

5. Procédé selon l'une des revendications précédentes caractérisé en ce que :
a) on prélève le volume de moût par aspiration au moyen d'une pompe (1), et on délivre ledit volume vers une cuve de stockage (5),
b) on évacue le moût hors de la cuve (5) vers le récipient (15), sous pression d'air comprimé délivré à l'intérieur de ladite cuve, et on clarifie ledit moût, lors de cette évacuation, au moyen d'une crépine de filtration (9),
c) on récupère le moût "décoloré" par la résine dans deux chambres de dosage (28) présentant chacune un volume déterminé.

6. Procédé selon la revendication 5 caractérisé en ce que l'on procède à une filtration supplémentaire du moût au moyen de couches accolées de matériaux présentant une porosité décroissante, selon le sens d'écoulement du moût, disposés dans le récipient (15) en amont de la résine.

7. Procédé selon l'une des revendications 5 ou 6 caractérisé en ce qu'aux étapes (d) et (j) on réalise les échantillons en laissant s'écouler, par gravité, les deux doses de moût récupérées dans les chambres de dosage (28) vers deux enceintes de mesure (55, 56), et en délivrant vers chacune desdites enceintes les doses prédéterminées de réactifs.

8. Procédé selon la revendication 7 caractérisé en ce que parallèlement aux étapes (d), (j), d'une part, on procède à un lavage et à un séchage de la cuve de stockage (5) et des canalisations (2) de pompage de moût, et d'autre part, on évacue le récipient (15) utilisé pour décolorer le moût.

9. Procédé selon l'une des revendications 7 ou 8 caractérisé en ce que l'on procède à un chauffage des échantillons se trouvant dans les enceintes de mesure (55, 56) de façon à amener ces derniers à une température sensiblement égale à 37° C.

10. Procédé selon l'une des revendications précédentes caractérisé en ce qu'à l'étape (d) on réalise un échantillon composé d'un centimètre cube de moût décoloré, de 0,6 centimètre cube de syringaldazine à 60 mg/litre et de 1,4 centimètre cube de solution tampon constituée d'acétate de sodium 0,1 M à pH 5.

11. Procédé selon la revendication 3 caractérisé en ce qu'à l'étape (j2) l'on réalise un échantillon composé d'un centimètre cube de moût décoloré, d'un centimètre cube d'acide sulfurique, de 0,5 centimètre cube de thiodène et d'une dose de 0,5 centimètre cube de solution iodée.

12. Procédé selon la revendication 11 caractérisé en ce que l'on utilise une solution iodée composée d'un mélange d'iodure et d'iodate de potassium.

13. Procédé selon l'une des revendications 7, 8 ou 9 caractérisé en ce que, préalablement aux mesures, on éloigne les enceintes de mesure (55, 56) des chambres de dosage (28), on obture lesdites enceintes et on procède à une agitation de ces dernières.

14. Procédé selon les revendications 8 et 13 prises ensemble, caractérisé en ce que, en parallèle avec les étapes de mesure et après éloignement des enceintes de mesure (55, 56) :
- on dispose en lieu et place du récipient (15) évacué, un conduit (21) apte à assurer une communication entre la cuve de stockage (5) et les chambres de dosage (28),
- on procède au lavage et au séchage, d'une part, des canalisations (10) de liaison entre lesdites cuve de stockage et chambres de dosage, et d'autre part, des chambres de dosage (28).

15. Procédé selon la revendication 13 caractérisé en ce que, après réalisation des mesures, on vidange par gravité les enceintes de mesure (55, 56), et on procède au lavage et au séchage desdites enceintes.

16. Procédé selon l'une des revendications précédentes caractérisé en ce que l'on utilise, comme résine, de la polyvinylpolypyrrolidone (P.V.P.P.) additionnée de sable destiné à améliorer sa porosité et la percolation.

17. Dispositif de mesure de l'activité Laccase en vue de la mise en oeuvre du procédé conforme à l'une des revendications 1 à 16 caractérisé en ce qu'il comprend en combinaison :
- des moyens de prélèvement (1) adaptés pour collecter une quantité prédéterminée de moût,
- des moyens (9) de clarification du moût prélevé,
- un récipient (15) doté d'une entrée de fluide et d'une sortie de fluide et renfermant une résine apte à adsorber les anthocyanes et les tanins, ledit récipient étant apte à laisser s'écouler le moût par la sortie de fluide et à retenir la résine,
- des moyens (8) de mise en pression adaptés pour injecter sous pression le moût clarifié au travers du récipient (15) renfermant la résine,
- des moyens de stockage (46) de produits chimiques contenant notamment de la syringaldazine, une solution tampon, et une solution iodée,
- des premiers moyens de dosage (26, 46) adaptés pour réaliser un premier échantillon constitué de doses prédéterminées de moût décoloré, de syringaldazine et de solution tampon,
- des deuxièmes moyens de dosage (26, 46) adaptés pour réaliser un deuxième échantillon constitué de doses prédéterminées de moût décoloré et de solution iodée,
- des moyens (55) de mesure colorimétrique adaptés pour effectuer des mesures de densité optique du premier échantillon, à une longueur d'onde où la couleur rouge absorbe,
- des moyens de mesure (56) adaptés pour détecter la présence éventuelle de dioxyde de soufre dans le deuxième échantillon,
- des moyens de transmission aptes à transmettre des signaux représentatifs des résultats des mesures effectuées par les moyens de mesure (55, 56) précitées,
- des moyens de canalisation (2, 10, 44, 45) adaptés pour assurer le transfert du moût et des échantillons vers les moyens de mesure (55, 56),
- et une unité centrale reliée aux moyens de transmission et adaptée, d'une part, pour activer successivement les moyens de prélèvement (1), les moyens de mise en pression (8), les moyens de dosage (26, 46) et les moyens de mesure (55, 56) et d'autre part, pour :
- commander à intervalle de temps déterminé, deux mesures colorimétriques du premier échantillon et calculer l'activité Laccase à partir des deux signaux délivrés par les moyens de mesure colorimétrique, puis délivrer un signal représentatif de ladite activité Laccase vers des moyens d'affichage,
- délivrer un signal d'inhibition de la mesure de l'activité Laccase, si la concentration en dioxyde de soufre dans le deuxième échantillon est supérieure à une valeur prédéterminée.

18. Dispositif selon la revendication 17 caractérisé en ce que :
- les moyens de stockage de produits chimiques contiennent également un indicateur coloré tel que du thiodène, et un acide tel que l'acide sulfurique,
- les deuxièmes moyens de dosage (24, 46) sont adaptés pour réaliser un échantillon constitué de doses prédéterminées de moût décoloré, d'indicateur coloré, d'acide et de solution iodée,
- les moyens de mesure (56) pour la détection de dioxyde de soufre sont des moyens de mesure colorimétrique (70) à une longueur d'onde où la couleur bleue absorbe,
- l'unité centrale est adaptée pour commander une première mesure colorimétrique à blanc, d'étalonnage, des moyens de mesure colorimétrique (56), commander le transfert de l'échantillon vers lesdits moyens de mesure, commander une deuxième mesure colorimétrique, et calculer la concentration en dioxyde de soufre par mesure de la valeur correspondant à la différence entre les deux mesures effectuées.

19. Dispositif selon l'une des revendications 17 ou 18 caractérisé en ce que :
- les moyens de prélèvement comprennent des moyens de pompage (1) aptes à amener le long d'une canalisation de pompage (2) une quantité prédéterminée de moût à l'intérieur d'une cuve de stockage (5) dotée d'une ouverture de remplissage (5a) et d'une ouverture d'évacuation (5b),
- les moyens de clarification comprennent une crépine de filtration (9) disposée au niveau de l'ouverture d'évacuation (5b) de la cuve de stockage (5).

20. Dispositif selon la revendication 19, caractérisé en ce qu'il comprend des moyens de filtration additionnels composés de couches accolées de matériaux de porosité décroissante, disposées dans chaque récipient (15), en amont de la résine.

21. Dispositif selon l'une des revendications 19 ou 20, caractérisé en ce que la cuve de stockage (5) et la canalisation de pompage (2) sont reliées à des moyens (13, 14) d'alimentation en eau de rinçage et en air de séchage aptes à permettre le lavage et le séchage desdites cuve et canalisation.

22. Dispositif selon l'une des revendications 19 à 21, caractérisé en ce que les moyens de mise en pression comprennent une canalisation (7) d'alimentation en air comprimé raccordée à la cuve de stockage (5) en vue de permettre une mise en pression de cette dernière entraînant une évacuation sous pression du moût au travers de l'ouverture d'évacuation (5b).

23. Dispositif selon l'une des revendications 17 à 22, caractérisé en ce que les premier et deuxième moyens de dosage comprennent un doseur de moût (26) doté de deux chambres de dosage (28) de volume prédéterminé, et des moyens de déplacement (30) desdites chambres entre une position de remplissage par le moût décoloré sortant du récipient (15), et une position de vidange vers les moyens (55, 56) de mesure respectifs de chaque échantillon.

24. Dispositif selon la revendication 23, caractérisé en ce que le doseur (26) comporte, pour chaque échantillon :
- un fourreau (35) présentant deux tronçons de sections différentes : un tronçon amont doté d'une ouverture (36) de communication avec un récipient (15), et un tronçon aval (35a) de section supérieure à celle du tronçon amont, doté d'une ouverture de communication avec les moyens de mesure (55, 56),
- un tiroir (37) de section conjuguée de celle du tronçon amont du fourreau (35), comportant un tronçon intermédiaire de section inférieure à sa section courante, formant une chambre de dosage annulaire (28) à l'intérieur dudit tronçon amont,
- des moyens d'étanchéité (38, 39) solidaires du tiroir (37) et disposés de part et d'autre de la chambre de dosage (28),
- les moyens de déplacement (30) étant adaptés pour déplacer les tiroirs (37) entre une position de remplissage où les chambres de dosage (28) sont disposées dans les tronçons amont desdits fourreaux en regard de l'ouverture de ces derniers, et une position de vidange où lesdites chambres de stockage sont disposées dans les tronçons aval (35a) de façon à permettre l'évacuation du moût au travers des ouvertures desdits tronçons aval.

25. Dispositif selon la revendication 24, caractérisé en ce que le doseur (26) comporte un bloc (29) d'évacuation du moût comprenant :
- deux réservoirs (44) agencés pour être alimentés respectivement par les premier et deuxième moyens de dosage (46) en réactifs, et dotés d'une sortie d'évacuation des échantillons vers les moyens de mesure (55, 56) correspondants,
- deux conduits (45) agencés pour venir en continuité chacun d'un fourreau (35), de façon à relier l'ouverture du tronçon aval (35a) de ce dernier à un réservoir (44).

26. Dispositif selon la revendication 25, caractérisé en ce que le doseur (26) et le bloc d'évacuation (29) sont agencés pour permettre une évacuation par gravité des produits vers les moyens de mesure (55, 56).

27. Dispositif selon l'une des revendications 25 ou 26, caractérisé en ce que les moyens de mesure comprennent deux enceintes de mesure (55, 56) dotées chacune d'une ouverture de remplissage agencée pour venir en communication avec une sortie d'évacuation du bloc d'évacuation (29) du doseur (26).

28. Dispositif selon la revendication 27, caractérisé en ce qu'il comprend :
- des moyens (58, 59) de déplacement en translation des moyens de mesure (55, 56) aptes à les déplacer entre une position avancée de remplissage à l'aplomb du bloc d'évacuation (29) du doseur (26), et une position reculée de mélange où les ouvertures de remplissage sont fermées par un obturateur fixe (60),
- des moyens de rotation (61, 63-66) des moyens de mesure (55, 56) autour de leur axe de translation.

29. Dispositif selon la revendication 28, caractérisé en ce qu'il comprend des moyens de rinçage (67) et de séchage (68) des enceintes de mesure (55, 56), disposés de façon à assurer le lavage et le séchage de ces dernières dans leur position avancée, après une rotation de 180° par rapport à leur position de remplissage.

30. Dispositif selon l'une des revendications 27 à 29, caractérisé en ce que les moyens de mesure comportent :
- une enceinte de mesure (56) associée à une diode électro-luminescente apte à permettre des mesures à une longueur d'onde égale à 635 nm pour la détection de dioxyde de soufre,
- une enceinte de mesure (55) associée à une lampe incandescente (57) et à un filtre aptes à permettre des mesures à une longueur d'onde égale à 530 nm pour la mesure de l'activité Laccase,
- des moyens de régulation de l'intensité de la lampe incandescente (57) aptes à permettre de maintenir la température des enceintes de mesure (55, 56) à une valeur sensiblement égale à 37° C.

31. Dispositif selon l'une des revendications 25 à 30, caractérisé en ce que les moyens de dosage en réactifs comprennent, pour chaque réactif, une pipette de dosage (46) dotée d'une sortie de liquide reliée à un des réservoirs (44), d'un piston (46a) associé à des moyens (49-53) d'actionnement mécanique dudit piston, et d'une entrée de liquide reliée à un des moyens de stockage en réactif.

32. Dispositif selon l'une des revendications 17 à 31, caractérisé en ce qu'il comprend :
- un magasin (16) apte à loger une pluralité de récipients (15),
- des moyens (19, 20) de préhension individuelle d'un récipient (15) dans le magasin (16),
- et des moyens (22) de déplacement des moyens de préhension (19, 20) aptes à déplacer ces derniers entre une position de préhension d'un récipient (15), et une position où ledit récipient se trouve interposé entre les moyens de prélèvement et les moyens de dosage.

33. Dispositif selon la revendication 32 et les revendications 21 et 23 prises ensemble, caractérisé en ce qu'il comprend un récipient vide (21) porté par les moyens de préhension (19, 20) et agencé pour se trouver interposé entre la cuve de stockage (5) et le doseur (26), dans la position de préhension des moyens de déplacement (22), de façon à permettre un lavage et un séchage du doseur (26) par l'intermédiaire des moyens d'alimentation en eau de rinçage (13) et en air de séchage (14) de ladite cuve.

34. Dispositif selon la revendication 33, caractérisé en ce que :
- le magasin (16) présente une forme en entonnoir de longueur conjuguée de la longueur d'un récipient,
- les moyens de préhension comprennent deux rails (19) dotés en regard d'une encoche transversale (20) apte à loger un récipient (15) et reliés, à distance de ladite première encoche, par un tube vide (21) de forme conjuguée d'un récipient (15) communiquant avec deux orifices de formes conjuguées ménagés dans lesdits rails.

35. Dispositif selon l'une des revendications 17 à 33, caractérisé en ce que chaque récipient (15) est constitué d'une cartouche comportant deux extrémités dotées de pastilles percées d'une perforation centrale apte à permettre une circulation du moût et, interposées entre lesdites pastilles et selon le sens d'écoulement du moût, au moins deux couches de matériau de filtration de porosité décroissante, une épaisseur de résine constituée de polyvinylpolypyrrolidone additionnée de sable, et au moins une couche de matériau apte à retenir la résine et à laisser s'écouler le moût.

## Claims

1. Process for measuring the laccase activity in must by the syringaldazine method, characterised in that it consists:
a) in extracting a predetermined volume of must,
b) in clarifying the extracted must and injecting the clarified must under pressure through a receiving unit (15) enclosing a resin capable of adsorbing anthocyans and tannins,
c) and in recovering two doses of a given volume of must "discoloured" by said resin,
d) in producing a sample consisting of one of the doses of must and of predetermined doses of syringaldazine and of buffer solution,
e) in subjecting the sample within a given period of time to two colorimetric measurements at a wavelength at which the colour red absorbs,
f) in delivering signals representative of the optical densities found in the course of the two colorimetric measurements,
g) in calculating the laccase activity as a function of the difference between the results of the two colorimetric measurements,
h) in delivering a signal representative of the laccase activity calculated to display means,
i) and, proceeding in parallel in relation to the aforementioned stages d), e), f), g) and h),
j) in producing a sample consisting of the other dose of discoloured must and of a predetermined dose of iodine solution and in checking for the possible presence of sulphur dioxide in the aforementioned sample,
k) and in delivering a signal inhibiting the result of the measurement of laccase activity if the sulphur dioxide concentration exceeds a predetermined threshold.

2. Process according to Claim 1 in which the colorimetric measurements are carried out at a wavelength equal to 530 nm.

3. Process according to one of Claims 1 or 2, characterised in that in stage (j) :
j1) a blind colorimetric calibration measurement is carried out at a wavelength at which the colour blue absorbs,
j2) a sample is produced consisting of a predetermined dose of discoloured must and of predetermined doses of a coloured indicator such as thiodene, an acid such as sulphuric acid and iodine,
j3) the sample is subjected to a colorimetric measurement at the wavelength at which the colour blue absorbs,
j4) the value corresponding to the difference in optical density between the two measurements is measured, and
(k) a signal inhibiting the result of the laccase-activity measurement is delivered if the measured value exceeds a predetermined threshold.

4. Process according to Claim 3, characterised in that in stage (j) colorimetric measurements are carried out at a wavelength equal to 665 nm.

5. Process according to one of the preceding claims, characterised in that:
a) the volume of must is extracted by suction with the aid of a pump (1) and said volume is supplied to a storage tank (5),
b) the must is evacuated from the tank (5) to the receiving unit (15) subject to the pressure of compressed air fed into said tank, and in the process of this evacuation said must is clarified with the aid of a filtering basket (9),
c) the must "discoloured" by the resin is recovered in two dosing chambers (28), each of which has a given volume.

6. Process according to Claim 5, characterised in that supplementary filtration of the must is effected by means of consecutively joined layers of materials having decreasing porosity in the direction of flow of the must, said materials being disposed within the receiving unit (15) upstream of the resin.

7. Process according to one of Claims 5 or 6, characterised in that in stages (d) and (j) the samples are produced by allowing the two doses of must recovered within the dosing chambers (28) to flow by gravity to two metering cells (55, 56) and in delivering to each of said cells the predetermined doses of reagent.

8. Process according to Claim 7, characterised in that proceeding in parallel with the stages (d) and (j) the storage tank (5) and the must-pumping conduits (2) are, on the one hand, washed and dried while, on the other hand, the receiving unit (15) used for discolouring the must is evacuated.

9. Process according to one of Claims 7 or 8, characterised in that the samples in the measuring cells (55, 56) are heated so as to raise the temperature of the latter to a value substantially equal to 37° C.

10. Process according to one of the preceding claims, characterised in that in stage (d) a sample is produced consisting of one cubic centimetre of discoloured must, 0.6 cubic centimetres of syringaldazine at 60 mg/litre and 1.4 cubic centimetres of buffer solution consisting of sodium acetate (0.1 M at pH 5).

11. Process according to Claim 3, characterised in that in stage (j2) a sample is produced consisting of one cubic centimetre of discoloured must, one cubic centimetre of sulphuric acid, 0.5 cubic centimetre of thiodene and a dose of 0.5 cubic centimetre of iodine solution.

12. Process according to Claim 11, characterised in that use is made of an iodine solution consisting of a mixture of potassium iodide and potassium iodate.

13. Process according to one of Claims 7, 8 or 9, characterised in that prior to carrying out the measurements the measuring cells (55, 56) are removed from the dosing chambers (28), whereupon said cells are blocked and subjected to agitation.

14. Process according to Claims 8 and 13 jointly, characterised in that, in parallel with the measuring stages and after removal of the measuring cells (55, 56) :
- a duct (21) capable of ensuring communication between the storage tank (5) and the dosing chambers (28) is provided instead and in place of the evacuated receiving unit (15),
- on the one hand, the conduits (10) linking said storage tank with the dosing chambers, and, on the other hand, the dosing chambers (28) are subjected to washing and drying.

15. Process according to Claim 13, characterised in that after carrying out the measurements the measuring cells (55, 56) are drained by gravity and said cells are subjected to washing and drying.

16. Process according to one of the preceding claims, characterised in that by way of resin use is made of polyvinyl polypyrrolidone (P.V.P.P) to which sand has been added with a view to improving its porosity and the percolation.

17. Device for measuring the laccase activity with a view to implementing the process according to one of Claims 1 to 16, characterised in that it comprises in combination:
- extraction means (1) so adapted as to collect a predetermined quantity of must,
- means (9) for clarifying the must which has been extracted,
- a receiving unit (15) provided with a fluid inlet and a fluid outlet and enclosing a resin capable of adsorbing anthocyans and tannins, said receiving unit being capable of allowing the must to flow out through the fluid outlet and to retain the resin,
- pressurising means (8) so adapted as to inject the clarified must under pressure through the receiving unit (15) enclosing the resin,
- means (46) for storing chemical products containing in particular syringaldazine, a buffer solution and an iodine solution,
- first dosing means (26, 46) so adapted as to produce a first sample consisting of predetermined doses of discoloured must, syringaldazine and buffer solution,
- second dosing means (26, 46) so adapted as to produce a second sample consisting of predetermined doses of discoloured must and iodine solution,
- means (55) for colorimetric measurement so adapted as to effect measurements of the optical density of the first sample at a wavelength at which the colour red absorbs,
- measuring means (56) so adapted as to detect the possible presence of sulphur dioxide in the second sample,
- transmission means capable of transmitting signals representative of the results of the measurements carried out by means of the aforementioned measuring means (55, 56),
- channeling means (2, 10, 44, 45) so adapted as to ensure the transfer of the must and of the samples to the measuring means (55, 56),
- and a central unit connected to the transmission means and so adapted as to, on the one hand, successively activate the extracting means (1), the pressurising means (8), the dosing means (26, 46) and the measuring means (55, 56) and, on the other hand, so as to:
- control, after a given period of time, two colorimetric measurements of the first sample and calculate the laccase activity from the two signals delivered by the colorimetric measuring means, and then deliver a signal representative of said laccase activity to display means,
- deliver a signal inhibiting the measurement of the laccase activity if the sulphur-dioxide concentration in the second sample exceeds a predetermined value.

18. Device according to Claim 17, characterised in that:
- the means for storing chemical products also contain a coloured indicator such as thiodene and an acid such as sulphuric acid,
- the second dosing means (26, 46) are so adapted as to produce a sample consisting of predetermined doses of discoloured must, coloured indicator, acid and iodine solution,
- the measuring means (56) for detecting sulphur dioxide are means for colorimetric measuring (70) at a wavelength at which the colour blue absorbs,
- the central unit is so adapted as to control a first blind colorimetric calibration measurement and means for colorimetric measurement (56), to control the transfer of the sample to said measuring means, to control a second colorimetric measurement and to calculate the sulphur-dioxide concentration by measuring the value corresponding to the difference between the two measurements effected.

19. Device according to one of Claims 17 or 18, characterised in that:
- the extraction means comprise pumping means (1) capable of feeding a predetermined quantity of must along a pumping conduit (2) into a storage tank (5) provided with a feed opening (5a) and a discharge opening (5b),
- the clarification means comprise a filtering basket (9) disposed in the region of the discharge opening (5b) of the storage tank (5).

20. Device according to Claim 19, characterised in that it comprises additional filtration means consisting of conjoined layers of materials with decreasing porosity disposed in each receiving unit (15) upstream of the resin.

21. Device according to one of Claims 19 or 20, characterised in that the storage tank (5) and the pumping conduit (2) are connected to means (13, 14) for supplying rinsing water and drying air capable of enabling washing and drying of said tank and conduit.

22. Device according to one of Claims 19 to 21, characterised in that the pressurising means comprise a conduit (7) for supplying compressed air, said conduit being connected to the storage tank (5) with a view to enabling pressurisation of the latter so as to bring about discharge under pressure of the must through the discharge opening (5b).

23. Device according to one of Claims 17 to 22, characterised in that the first and second dosing means comprise a must-dosing device (26) provided with two dosing chambers (28) of predetermined volume and means (30) for displacing said chambers between a feeding position in which they are supplied with the discoloured must emanating from the receiving unit (15) and a position in which they are drained towards the respective measuring means (55, 56) for each sample.

24. Device according to Claim 23, characterised in that the dosing device (26) comprises for each sample:
- a sleeve (35) having two sections of different cross-section: an upstream section provided with an opening (36) for communication with a receiving unit (15) and a downstream section (35a), the cross-section of which is larger than that of the upstream section, said downstream section having an opening for communication with the measuring means (55, 56),
- a slide valve (37) having a cross-section matching that of the upstream section of the sleeve (35) whereby said slide valve comprises an intermediate section, the cross-section of which is smaller than its standard cross-section so as to form an annular dosing chamber (28) within said upstream section,
- sealing means (38, 39) firmly connected to the slide valve (37) and arranged on either side of the dosing chamber (28),
- whereby the displacing means (30) are so adapted as to displace the slide valves (37) between a feeding position in which the dosing chambers (28) are located within the upstream sections of said sleeves opposite the openings in the latter and a drainage position in which said storing chambers are located within the downstream sections (35a) so as to enable discharge of the must through the openings in said downstream sections.

25. Device according to Claim 24, characterised in that the dosing unit (26) comprises a block (29) for discharging the must, said dosing unit comprising:
- two reservoirs (44) so arranged as to be supplied with reagents by the first and second dosing means (46), respectively, and provided with an outlet for discharging the samples to corresponding measuring means (55, 56),
- two ducts (45), each of them being arranged in extension of a sleeve (35) so as to connect the opening of the downstream section (35a) of said sleeve to a reservoir (44).

26. Device according to Claim 25, characterised in that the dosing unit (26) and the discharge block (29) are so arranged as to enable discharge by gravity of the products to the measuring means (55, 56).

27. Device according to one of Claims 25 or 26, characterised in that the measuring means comprise two measuring cells (55, 56), each of which is provided with a feed opening so arranged as to communicate with a discharge outlet of the discharge block (29) of the dosing unit (26).

28. Device according to Claim 27, characterised in that it comprises:
- means (58, 59) for translational displacement of the measuring means (55, 56), said means being capable of displacing said measuring means between an advanced feeding position vertically below the discharge block (29) of the dosing unit (26) and a retracted mixing position in which the feed openings are closed by a fixed blocking element (60),
- means (61, 63-66) for rotating the measuring means (55, 56) about their axis of translation.

29. Device according to Claim 28, characterised in that it comprises means for rinsing (67) and for drying (68) the measuring cells (55, 56), said means being so arranged as to ensure that said measuring cells are washed and dried in their advanced position after rotation by 180° in relation to their feeding position.

30. Device according to one of Claims 27 to 29, characterised in that the measuring means comprise:
- a measuring cell (56) associated with an electroluminescent diode capable of enabling measurements at a wavelength equal to 635 nm with a view to detecting sulphur dioxide,
- a measuring cell (55) associated with an incandescent lamp (57) and with a filter capable of enabling measurements at a wavelength equal to 530 nm for measuring the laccase activity,
- means for regulating the intensity of the incandescent lamp (57) capable of enabling maintenance of the temperature of the measuring cells (55, 56) at a value substantially equal to 37° C.

31. Device according to one of Claims 25 to 30, characterised in that the means for dosing reagents comprise, in respect of each reagent, a dosing pipette (46) provided with a liquid outlet connected to one of the reservoirs (44), a piston (46a) associated with means (49-53) for mechanical actuation of said piston and a liquid inlet connected to one of the means for storing reagent.

32. Device according to one of Claims 17 to 31, characterised in that it comprises:
- a magazine (16) capable of accommodating a plurality of receiving units (15),
- means (19, 20) for individual gripping of a receiving unit (15) in the magazine (16),
- and means (22) for displacing the gripping means (19, 20), said means (22) being capable of displacing the latter between a position in which they grip a receiving unit (15) and a position in which said receiving unit is interposed between the extraction means and the dosing means.

33. Device according to Claim 32 and Claims 21 and 23 jointly, characterised in that it comprises an empty receiving unit (21) carried by the gripping means (19, 20) and so arranged as to be interposed between the storage tank (5) and the dosing unit (26) in the gripping position of the displacing means (22) in order to enable washing and drying of the dosing unit (26) via means for supplying said tank with rinsing water (13) and drying air (14).

34. Device according to Claim 33, characterised in that:
- the magazine (16) has the shape of a funnel, the length of which matches the length of a receiving unit,
- the gripping means comprise two rails (19) provided opposite a transverse recess (20) capable of accommodating a receiving unit (15) and connected at a distance from said first recess by a vacant tube (21), the shape of which matches that of a receiving unit (15) whereby said tube communicates with two apertures of matching shape provided within said rails.

35. Device according to one of Claims 17 to 33, characterised in that each receiving unit (15) is constituted by a cartridge comprising two ends provided with end discs, said end discs having a central perforation capable of enabling circulation of the must and, interposed between said end discs and in the direction in which the must flows, at least two layers of filtration material of decreasing porosity, a layer of resin constituted by polyvinyl polypyrrolidone to which sand has been added and at least one layer of material capable of retaining the resin while allowing the must to flow out.

## Patentansprüche

1. Verfahren zum Messen der Laccase-Aktivität in Most nach der Syringaldazinmethode, dadurch gekennzeichnet, daß es darin besteht:
a) eine vorbestimmte Mostmenge zu entnehmen,
b) den entnommenen Most zu klären und den geklärten Most unter Druck durch eine Aufnahmeeinheit (15) zu injizieren, wobei die besagte Aufnahmeeinheit ein Harz enthält, das so beschaffen ist, daß es die Anthocyane und die Tannine adsorbiert,
c) und zwei Dosen vorbestimmter Menge des durch das besagte Harz "verfärbten" Mosts zurückzugewinnen,
d) eine sich aus einer der Dosen von Most und vorbestimmten Dosen von Syringaldazin und Pufferlösung zusammensetzende Probe herzustellen,
e) innerhalb einer vorbestimmten Zeitspanne zwei kolorimetrische Messungen der Probe bei einer Wellenlänge vorzunehmen, bei der die Farbe rot absorbiert,
f) für die in Zuge der beiden kolorimetrischen Messungen bestimmten Schwärzungsgrade repräsentative Signale zu liefern,
g) die Laccase-Aktivität in Abhängigkeit von dem Unterschied zwischen den Ergebnissen der beiden kolorimetrischen Messungen zu berechnen,
h) einem Sichtgerät ein für die berechnete Laccase-Aktivität repräsentatives Signal zu liefern,
i) und parallel zu den vorstehend genannten Stufen d), e), f), g) und h),
j) eine Probe herzustellen, die sich aus der anderen Dosis von verfärbtem Most sowie einer vorbestimmten Dosis von Jodlösung zusammensetzt, und nachzuprüfen, ob die besagte Probe Schwefeldioxid enthält,
k) und ein Signal zum Sperren des Ergebnisses der Laccase-Aktivitätsmessung zu liefern, wenn die Schwefeldioxidkonzentration einen vorbestimmten Schwellwert überschreitet.

2. Verfahren nach Anspruch 1, bei dem die kolorimetrischen Messungen bei einer Wellenlänge von 530 nm vorgenommen werden.

3. Verfahren nach einem der Ansprüche 1 oder 2, dadurch gekennzeichnet, daß in der Stufe (j):
j1) eine blinde kolorimetrische Eichmessung bei einer Wellenlänge vorgenommen wird, bei der die Farbe blau absorbiert,
j2) eine Probe hergestellt wird, die sich aus einer vorbestimmten Dosis von verfärbtem Most und vorbestimmten Dosen eines farbigen Indikators wie Thioden, einer Säure wie Schwefelsäure und Jod zusammensetzt,
j3) eine kolorimetrische Messung der Probe bei der Wellenlänge vorgenommen wird, bei der die Farbe blau absorbiert,
j4) der dem Unterschied zwischen den in den beiden Messungen bestimmten Schwärzungsgraden entsprechende Wert gemessen wird und
(k) ein Signal zum Sperren des Ergebnisses der Laccase-Aktivitätsmessung zu liefern, wenn der gemessene Wert einen vorbestimmten Schwellwert überschreitet.

4. Verfahren nach Anspruch 3, dadurch gekennzeichnet, daß in Stufe (j) kolorimetrische Messungen bei einer Wellenlänge von 665 nm vorgenommen werden.

5. Verfahren nach einem der vorstehenden Ansprüche, dadurch gekennzeichnet, daß
a) die Mostmenge durch Ansaugen mit Hilfe einer Pumpe (1) entnommen und die besagte Menge einem Vorratsbehälter (5) zugeführt wird,
b) der Most von dem Behälter (5) unter dem Druck von in den besagten Behälter eingeführter Druckluft in Richtung der Aufnahmeeinheit (15) abgegeben und der besagte Most im Zuge dieser Abgabe mit Hilfe eines Filtrierkorbes (9) geklärt wird,
c) der durch das Harz "verfärbte" Most in zwei Dosierkammern (28) zurückgewonnen wird, wobei jede der besagten Kammern ein bestimmtes Volumen aufweist.

6. Verfahren nach Anspruch 5, dadurch gekennzeichnet, daß eine zusätzliche Filterung des Mosts mit Hilfe von aneinander angefügten Schichten von Materialien mit in der Mostablaufrichtung abnehmender Porosität stattfindet, wobei die besagten Materialien in der Aufnahmeeinheit (15) stromaufwärts von dem Harz angeordnet sind.

7. Verfahren nach einem der Ansprüche 5 oder 6, dadurch gekennzeichnet, daß in den Stufen (d) und (j) Proben hergestellt werden, indem man die beiden in den Dosierkammern (28) zurückgewonnenen Mostdosen durch Schwerkraft zwei Meßzellen (55, 56) zufließen läßt, und jeder der besagten Zellen vorbestimmte Reagenziendosen zuführt.

8. Verfahren nach Anspruch 7, dadurch gekennzeichnet, daß parallel zu den Stufen (d), (j) einerseits Waschung und Trocknung des Vorratsbehälters (5) und der Mostpumpleitungen (2) stattfindet und andererseits die zum Verfärben des Mostes verwendete Aufnahmeeinheit (15) entleert wird.

9. Verfahren nach einem der Ansprüche 7 oder 8, dadurch gekennzeichnet, daß die in den Meßzellen (55, 56) befindlichen Proben erwärmt werden, um sie auf eine im wesentlichen 37° C betragende Temperatur zu bringen.

10. Verfahren nach einem der vorstehenden Ansprüche, dadurch gekennzeichnet, daß in Stufe (d) eine Probe hergestellt wird, die sich aus einem Kubikzentimeter von verfärbtem Most, 0,6 Kubikzentimeter Syringaldazin mit einer Konzentration von 60 mg/Liter und 1,4 Kubikzentimeter aus Natriumacetat (0,1 M bei pH 5) bestehender Pufferlösung zusammensetzt.

11. Verfahren nach Anspruch 3, dadurch gekennzeichnet, daß in der Stufe (j2) eine Probe hergestellt wird, die sich aus einem Kubikzentimeter von verfärbtem Most, einem Kubikzentimeter Schwefelsäure, 0,5 Kubikzentimeter Thioden und einer Dosis von 0,5 Kubikzentimeter Jodlösung zusammensetzt.

12. Verfahren nach Anspruch 11, dadurch gekennzeichnet, daß von einer Jodlösung Gebrauch gemacht wird, die sich aus einem Gemisch von Kaliumjodid und Kaliumjodat zusammensetzt.

13. Verfahren nach einem der Ansprüche 7, 8 oder 9, dadurch gekennzeichnet, daß vor den Messungen die Meßzellen (55, 56) aus den Dosierkammern (28) entfernt und die besagten Zellen abgedichtet und schließlich gerüttelt werden.

14. Verfahren nach den Ansprüchen 8 und 13 gemeinsam, dadurch gekennzeichnet, daß parallel zu den Meßstufen und nach Entfernung der Meßzellen (55, 56) :
- anstelle der entleerten Aufnahmeeinheit (15) eine Rohrleitung (21) angeordnet wird, die so beschaffen ist, daß sie Kommunikation zwischen dem Vorratsbehälter (5) und den Dosierkammern (28) gewährleistet,
- die Leitungsrohre (10) zur Verbindung des besagten Vorratsbehälters mit den Dosierkammern einerseits und die Dosierkammern (28) andererseits gewaschen und getrocknet werden.

15. Verfahren nach Anspruch 13, dadurch gekennzeichnet, daß nach Durchführung der Messungen die Meßzellen (55, 56) durch Schwerkraft entleert und die besagten Zellen gewaschen und getrocknet werden.

16. Verfahren nach einem der vorstehenden Ansprüche, dadurch gekennzeichnet, daß als Harz von Polyvinylpolypyrrolidon (P.V.P.P.) mit einem Zusatz von Sand zwecks Verbesserung seiner Porosität und der Perkolation Gebrauch gemacht wird.

17. Vorrichtung zum Messen der Laccase-Aktivität zwecks Durchführung des Verfahrens nach einem der Ansprüche 1 bis 16, dadurch gekennzeichnet, daß sie in Verbindung folgende Teile umfaßt:
- Entnahmemittel (1), die zum Erfassen einer vorbestimmten Mostmenge konstruiert sind,
- Mittel (9) zum Klären des entnommenen Mosts,
- eine Aufnahmeeinheit (15) mit einem
Flüssigkeitseingang und einem Flüssigkeitsausgang, die ein Harz enthält, das so beschaffen ist, daß es Anthocyane und Tannine adsorbiert, wobei die besagte Aufnahmeeinheit so beschaffen ist, daß sie den Most durch den Flüssigkeitsausgang ablaufen läßt und das Harz zurückbehält,
- Unterdrucksetzungsmittel (8), die so konstruiert sind, daß sie den geklärten Most unter Druck durch die das Harz enthaltende Aufnahmeeinheit (15) hindurch einspritzen,
- Mittel (46) zur Speicherung chemischer Produkte, die insbesondere Syringaldazin, eine Pufferlösung und eine Jodlösung enthalten,
- erste Dosiermittel (26, 46), die so konstruiert sind, daß sie eine erste vorbestimmte Dosen von verfärbtem Most, Syringaldazin und Pufferlösung umfassende Probe erzeugen,
- zweite Dosiermittel (26, 46), die so konstruiert sind, daß sie eine zweite vorbestimmte Dosen von verfärbtem Most und Jodlösung umfassende Probe erzeugen,
- kolorimetrische Meßmittel (55) zur Messung des Schwärzungsgrades der ersten Probe bei einer Wellenlänge, bei der die Farbe rot absorbiert,
- Meßmittel (56), die so konstruiert sind, daß sie ggf. das Vorhandensein von Schwefeldioxid in der zweiten Probe nachweisen,
- Übertragungsmittel, die so beschaffen sind, daß sie für die Ergebnisse der durch die vorstehend genannten Meßmittel (55, 56) bewirkten Messungen repräsentative Signale übertragen,
- Leitmittel (2, 10, 44, 45), die so konstruiert sind, daß sie die Übertragung des Mosts und der Proben an die Meßmittel (55, 56) gewährleisten,
- und eine Zentraleinheit, die mit den Übertragungsmitteln in Verbindung steht und einerseits so konstruiert ist, daß sie die Entnahmemittel (1), die Unterdrucksetzungsmittel (8), die Dosiermittel (26, 46) und die Meßmittel (55, 56) der Reihe nach betätigt und andererseits:
- innerhalb einer vorbestimmten Zeitspanne zwei kolorimetrische Messungen der ersten Probe steuert und die Laccase-Aktivität auf Grund der beiden durch die Mittel für kolorimetrische Messungen gelieferten Signale berechnet, worauf sie ein für die besagte Laccase-Aktivität repräsentatives Signal an ein Sichtgerät liefert,
- ein Signal zum Sperren der Laccase-Aktivitätsmessung liefert, falls die Schwefeldioxidkonzentration in der zweiten Probe einen vorbestimmten Wert übersteigt.

18. Vorrichtung nach Anspruch 17, dadurch gekennzeichnet, daß:
- die Mittel zur Speicherung chemischer Produkte auch einen farbigen Indikator wie Thioden und eine Säure wie Schwefelsäure enthalten,
- die zweiten Dosiermittel (26, 46) so konstruiert sind, daß sie eine Probe erzeugen, die sich aus vorbestimmten Dosen von verfärbtem Most, gefärbtem Indikator, Säure und Jodlösung zusammensetzt,
- die Meßmittel (56) für den Nachweis von Schwefeldioxid Mittel für kolorimetrische Messung (70) bei einer Wellenlänge, bei der die Farbe blau absorbiert, sind,
- die Zentraleinheit so konstruiert ist, daß sie eine erste blinde kolorimetrische Eichmessung, Mittel für kolorimetrische Messung (56), die Übertragung der Probe an die besagten Meßmittel und eine zweite kolorimetrische Messung steuert und die Schwefeldioxidkonzentration durch Messen des dem Unterschied zwischen den beiden vorgenommenen Messungen entsprechenden Wertes berechnet.

19. Vorrichtung nach einem der Ansprüche 17 oder 18, dadurch gekennzeichnet, daß:
- die Entnahmemittel Pumpmittel (1) umfassen, die so beschaffen sind, daß sie eine vorbestimmte Mostmenge entlang einer Pumpleitung (2) in einen Vorratsbehälter (5) befördern, der mit einer Eingabeöffnung (5a) und einer Ablauföffnung (5b) versehen ist,
- die Klärmittel einen Filtrationskorb (9) umfassen, der im Bereich der Ablauföffnung (5b) des Vorratsbehälters (5) angeordnet ist.

20. Vorrichtung nach Anspruch 19, dadurch gekennzeichnet, daß sie zusätzliche Filtrationsmittel umfaßt, die sich aus miteinander verbundenen Materialschichten abnehmender Porosität zusammensetzen, wobei die besagten Schichten in jeder Aufnahmeeinheit (15) stromaufwärts von dem Harz angeordnet sind.

21. Vorrichtung nach einem der Ansprüche 19 oder 20, dadurch gekennzeichnet, daß der Vorratsbehälter (5) und die Pumpleitung (2) mit Mitteln (13, 14) für die Zufuhr von Spülwasser und Trockenluft in Verbindung stehen, die so beschaffen sind, daß sie das Waschen und Trocknen des besagten Behälters und der besagten Leitung gestatten.

22. Vorrichtung nach einem der Ansprüche 19 bis 21, dadurch gekennzeichnet, daß die Unterdrucksetzungsmittel eine Leitung (7) zum Zuführen von Druckluft umfassen, die mit dem Vorratsbehälter (5) in Verbindung steht, so daß dieser unter Druck gesetzt werden kann, was Abgabe des Mosts durch die Ablauföffnung (5b) hindurch zur Folge hat.

23. Vorrichtung nach einem der Ansprüche 17 bis 22, dadurch gekennzeichnet, daß das erste und das zweite Dosiermittel eine Mostdosiereinheit (26) umfassen, die mit zwei Dosierkammern (28) vorbestimmten Volumens versehen ist, sowie Mittel (30) zum Verlagern der besagten Kammern zwischen einer Eingabeposition, in der sie mit dem aus der Aufnahmeeinheit (15) ablaufenden verfärbtem Most gefüllt werden, und einer Entleerungsposition, in der sie in Richtung der betreffenden Meßmittel (55, 56) jeder Probe abgelassen werden.

24. Vorrichtung nach Anspruch 23, dadurch gekennzeichnet, daß die Dosiereinheit (26) hinsichtlich jeder Probe folgende Teile umfaßt:
- eine Hülse (35) mit zwei Abschnitten unterschiedlichen Querschnitts, und zwar mit einem stromaufwärts befindlichen Abschnitt, der eine mit einer Aufnahmeeinheit (15) kommunizierende Öffnung (36) aufweist, und mit einem stromabwärts befindlichen Abschnitt (35a), dessen Querschnitt größer ist als der des stromaufwärts befindlichen Abschnitts und der mit einer mit den Meßmitteln (55, 56) kommunizierenden Öffnung versehen ist,
- einen Steuerschieber (37), dessen Querschnitt dem des stromaufwärts befindlichen Abschnitts der Hülse (35) entspricht, umfassend einen Zwischenabschnitt, dessen Querschnitt kleiner ist als sein regulärer Querschnitt, so daß er im Inneren des besagten stromaufwärts befindlichen Abschnitts eine ringförmige Dosierkammer (28) bildet,
- Abdichtmittel (38, 39), die fest mit dem Steuerschieber (37) verbunden und zu beiden Seiten der Dosierkammer (28) angeordnet sind,
- wobei die Verlagerungsmittel (30) so konstruiert sind, daß sie die Steuerschieber (37) zwischen einer Eingabeposition, in der die Dosierkammern (28) in den stromabwärts befindlichen Abschnitten der besagten Hülsen gegenüber den in diesen Hülsen vorgesehenen Öffnungen angeordnet sind, und einer Ablaufposition, in der die besagten Vorratskammern in den stromabwärts befindlichen Abschnitten (35a) angeordnet sind, so daß der Most durch die Öffnungen der besagten stromabwärts befindlichen Abschnitte hindurch ablaufen kann, verlagern.

25. Vorrichtung nach Anspruch 24, dadurch gekennzeichnet, daß die Dosiereinheit (26) einen Mostablaufblock (29) aufweist, der folgende Teile umfaßt:
- zwei Speicher (44), die so angeordnet sind, daß sie durch das erste bzw. das zweite Dosiermittel (46) mit Reagenzien versorgt werden und die mit einem Ausgang für Ablauf der Proben in Richtung der entsprechenden Meßmittel (55, 56) versehen sind,
- zwei Kanäle (45), von denen jeder so angeordnet ist, daß er die Fortsetzung einer Hülse (35) bildet, um die Öffnung des stromabwärts befindlichen Abschnitt (35a) der besagten Hülse mit einem Speicher (44) zu verbinden.

26. Vorrichtung nach Anspruch 25, dadurch gekennzeichnet, daß die Dosiereinheit (26) und der Ablaufblock (29) so angeordnet sind, daß sie schwerkraftbedingten Ablauf der Produkte in Richtung der Meßmittel (55, 56) gestatten.

27. Vorrichtung nach einem der Ansprüche 25 oder 26, dadurch gekennzeichnet, daß die Meßmittel zwei Meßzellen (55, 56) umfassen, von denen jede mit einer Eingabeöffnung versehen ist, wobei die besagte Eingabeöffnung so angeordnet ist, daß sie mit einem Ablaufausgang des Ablaufblocks (29) der Dosiereinheit (26) in Kommunikation gerät.

28. Vorrichtung nach Anspruch 27, dadurch gekennzeichnet, daß sie folgende Teile umfaßt:
- Mittel (58, 59) zu Translationsverlagerung der Meßmittel (55, 56), wobei die besagten Verlagerungsmittel so beschaffen sind, daß sie die Meßmittel zwischen einer vorgeschobenen Eingabeposition senkrecht unter dem Ablaufblock (29) der Dosiereinheit (26) und einer zurückgezogenen Mischposition verlagern, in der die Eingabeöffnung durch ein fest angeordnetes Blockierelement (60) abgeschlossen ist,
- Mittel (61, 63-66) zum Drehen der Meßmittel (55, 56) um deren Translationsachse.

29. Vorrichtung nach Anspruch 28, dadurch gekennzeichnet, daß sie Mittel (67) zum Spülen und Mittel (68) zum Trocknen der Meßzellen (55, 56) umfaßt, wobei die besagten Mittel so angeordnet sind, daß sie das Waschen bzw. Trocknen der Meßzellen in deren vorgeschobenen Position nach Drehung um 180° im Verhältnis zu deren Eingabeposition gewährleisten.

30. Vorrichtung nach einem der Ansprüche 27 bis 29, dadurch gekennzeichnet, daß die Meßmittel folgende Teile umfassen:
- eine mit einer elektrolumineszenten Diode in Verbindung stehende Meßzelle (56), die so beschaffen ist, daß sie zwecks Nachweis von Schwefeldioxid die Durchführung von Messungen bei einer Wellenlänge von 635 nm gestattet,
- eine mit einer Glühlampe (57) in Verbindung stehende Meßzelle (55) und ein Filter, die so beschaffen sind, daß sie zwecks Bestimmung der Laccase-Aktivität Messungen bei einer Wellenlänge von 530 nm gestatten,
- Mittel zur Regelung der Stromstärke der Glühlampe (57), mit deren Hilfe es möglich ist, die Temperatur der Meßzellen (55, 56) auf einem Wert von im wesentlichen 37° C zu erhalten.

31. Vorrichtung nach einem der Ansprüche 25 bis 30, dadurch gekennzeichnet, daß die Mittel zum Dosieren mit Reagenzien hinsichtlich jedes Reagens eine Dosierpipette (46) umfassen, die mit einem an einen der Speicher (44) angeschlossenen Flüssigkeitsauslaß, einem Kolben (46a), der mit Mitteln (49-53) für mechanische Betätigung des besagten Kolbens verbunden ist, und einem an eines der Reagenzspeichermittel angeschlossenen Flüssigkeitseingang versehen ist.

32. Vorrichtung nach einem der Ansprüche 17 bis 31, dadurch gekennzeichnet, daß sie folgende Teile umfaßt:
- ein Magazin (16), das so beschaffen ist, daß es eine Mehrzahl von Aufnahmeeinheiten (15) aufnimmt,
- und Mittel (19, 20) für individuelles Ergreifen einer Aufnahmeeinheit (15) in dem Magazin (16),
- Mittel (22) zum Verlagern der Greifmittel (19, 20), wobei die besagten Verlagerungsmittel so beschaffen sind, daß sie diese Greifmittel zwischen einer Greifposition, in der sie eine Aufnahmeeinheit (15) ergreifen, und einer Position, in der sich die besagte Aufnahmeeinheit zwischen den Entnahmemitteln und den Dosiermitteln befindet, verlagern.

33. Vorrichtung nach Anspruch 32 und den Ansprüchen 21 und 23 gemeinsam, dadurch gekennzeichnet, daß sie eine leere Aufnahmeeinheit (21) umfaßt, die durch die Greifmittel (19, 20) getragen und so angeordnet ist, daß sie sich zwischen dem Vorratsbehälter (5) und der Dosiereinheit (26) in der Greifposition der Verlagerungsmittel (22) befindet, so daß sie mit Hilfe der Mittel (13) für die Spülwasserzufuhr und Mitteln (14) für die Trockenluftzufuhr des besagten Behälters das Waschen bzw. Trocknen der Dosiereinheit (26) gestatten.

34. Vorrichtung nach Anspruch 33, dadurch gekennzeichnet, daß:
- das Magazin (16) trichterförmig ist und seine Länge der Länge einer Aufnahmeinheit entspricht,
- die Greifmittel zwei Schienen (19) umfassen, denen gegenüber sich eine Queraussparung (20) befindet, die so beschaffen ist, daß sie eine Aufnahmeeinheit (15) aufnimmt, wobei die besagten Schienen mit Abstand von der besagten ersten Aussparung durch ein leeres Rohr (21) von einer Aussparungseinheit (15) entsprechender Form verbunden ist, das mit zwei Öffnungen entsprechender Formen in den besagten Schienen in Verbindung steht.

35. Vorrichtung nach einem der Ansprüche 17 bis 33, dadurch gekennzeichnet, daß jede Aufnahmeeinheit (15) aus einer Patrone mit zwei Endscheiben umfassenden Enden besteht, wobei die besagten Endscheiben eine Mittelbohrung aufweisen, die so beschaffen ist, daß sie Umlauf des Mostes gestattet, und zwischen den besagten Endscheiben und in der Ablaufrichtung des Mostes mindestens zwei Schichten Filtermaterial abnehmender Porosität, eine Schicht von aus Polyvinylpolypyrrolidon bestehendem Harz mit einem Zusatz von Sand und mindestens einer Schicht aus einem Material besteht, die so beschaffen ist, daß sie das Harz zurückbehält und den Most abfließen läßt.
